# EUROPÄISCHE PATENTANMELDUNG

(11) **EP 0 893 165 A2**
(43) Veröffentlichungstag der Anmeldung: **27.01.1999**
(21) Anmeldenummer: 98110137.1
(22) Anmeldetag: 04.06.1998
(51) Int. Cl.: B05D 1/00, A61L 33/00, A61L 27/00

(54) **Bioaktive Beschichtung von Oberflächen unter Verwendung von Makroinitiatoren**

(30) Priorität: 28.06.1997 DE 19727555; 04.03.1998 DE 19809042
(71) Anmelder: HÜLS AKTIENGESELLSCHAFT, 45764 Marl (DE)
(72) Erfinder: Anders, Christine, Dr., 45721 Haltern (DE); Meier-Haack, Jochen, Dr., 01259 Dresden (DE); Steinert, Volker, Dr., 01099 Dresden (DE); Zschoche, Stefan, Dr., 01109 Dresden (DE); Jacobasch, Hans-Jörg, Prof. Dr., 01187 Dresden (DE)

(57) **Zusammenfassung**

Die Erfindung betrifft Verfahren zur bioaktiven Beschichtung der Oberfläche von Substraten, dadurch gekennzeichnet, daß man mindestens ein Vinylmonomer der allgemeinen Formel R-(A)ₐ (I), in der
R einen olefinisch ungesättigten organischen Rest mit der Wertigkeit a bedeutet,
A eine Carboxylgruppe -COOH, Schwefelsäuregruppe -OSO₂OH, Sulfonsäuregruppe -SO₃H, Phosphorsäuregruppe -OPO(OH)₂, Phosphonsäuregruppe -PO(OH)₂, Phosphorigsäuregruppe -OP(OH)₂, phenolische Hydroxylgruppe oder ein Salz einer der genannten Gruppen bezeichnet und
a für eine ganze Zahl von 1 bis einschließlich 6 steht.
strahleninduziert oder thermisch auf einer Substratoberfläche pfropfpolymerisiert, die zuvor mit einem Makroinitator mit Radikale bildenden Gruppen in Seitenketten des Polymergerüstes behandelt wurde; mit der Maßgabe, daß ein Monomer I, in dem A eine Carboxylgruppe -COOH oder ein Salz der Carboxylgruppe bedeutet, entweder mindestens einen weiteren Rest A mit einer anderen der für A genannten Bedeutungen enthält oder zusammen mit mindestens einem weiteren Monomeren I verwendet wird, in dem A eine andere der für A genannten Bedeutungen hat. Gegebenenfals wird bei der Beschichtung ein Vernetzer mitverwendet. Die Erfindung betrifft auch Erzeugnisse, deren Oberfläche ganz oder teilweise nach dem Verfahren beschichtet ist, und deren Verwendung im medizinischen oder biotechnischen oder im Hygienebereich.

## Beschreibung

Die Erfindung betrifft ein Verfahren zur bioaktiven Beschichtung der Oberfläche von Substraten, vorzugsweise von Kunststoff- (oder Polymer-) substraten unter Verwendung eines Makroinitiators. Eine wichtige Eigenschaft der erfindungsgemäß aufgebrachten Beschichtungen ist ihre gute Verträglichkeit im Kontakt mit Körperflüssigkeiten und Gewebe, insbesondere mit Blut. Je nach Funktionalität der Beschichtungsmonomeren bzw. nach dem Molverhältnis bestimmter funktioneller Gruppen in der Beschichtung werden die Oberflächen darüber hinaus bakterienabweisend und zellproliferationsinhibierend oder bakterienabweisend und zellproliferationsfördernd. Die Erfindung betrifft weiterhin Erzeugnisse mit derart beschichteten Oberflächen zur Verwendung für medizinische oder biotechnische Zwecke oder im Hygienebereich.

### 1. Stand der Technik

Die Ansiedelung und Vermehrung von Bakterien auf Oberflächen ist eine in der Regel unerwünschte Erscheinung, die häufig mit nachteiligen Folgen verbunden ist. So können in der Trinkwasser- und Getränketechnik Bakterienpopulationen zu einer gesundheitsgefährdenden Qualitätsminderung führen. Bakterien auf oder in Verpackungen bewirken häufig den Verderb von Lebensmitteln oder verursachen sogar Infektionen bei dem Verbraucher. In steril zu betreibenden biotechnischen Anlagen stellen systemfremde Bakterien ein erhebliches prozeßtechnisches Risiko dar. Solche Bakterien können mit Rohstoffen eingetragen werden oder bei mangelhafter Sterilisation in allen Anlageteilen zurückbleiben. Teile der Bakterienpopulation können sich durch Adhäsion dem normalen Flüssigkeitsaustausch beim Spülen und Reinigen entziehen und sich im System vermehren.

Weiterhin sind Bakterienansiedelungen in Wasseraufbereitungsanlagen (z.B. zur Entsalzung durch Membranen) oder auch in Behältern bekannt, die mit gelösten oder flüssigen unverdünnten organischen Substanzen gefüllt sind und für Bakterienpopulationen vorteilhafte Bedingungen aufweisen. Solche mikrobiellen Belegungen können in erheblichem Umfang zur Blockierung und/oder korrosiven Zerstörung der Anlage führen.

Besondere Bedeutung kommt dem Schutz vor Bakterienanhaftung und -ausbreitung in der Ernährung, der Pflege, hier insbesondere in der Altenpflege. und in der Medizin zu. Bei Massenbeköstigungen oder -ausschank existieren besonders dann erhebliche Risiken, wenn zur Vermeidung von Abfall von Einweggeschirr abgesehen wird und eine nur unzureichende Reinigung des Mehrweggeschirrs erfolgt. Die schädliche Ausbreitung von Bakterien in lebensmittelführenden Schläuchen und Rohren ist ebenso bekannt wie die Vermehrung in Lagerbehältern sowie in Textilien in feuchter und warmer Umgebung, z.B. in Bädern. Solche Einrichtungen sind bevorzugte Lebensräume für Bakterien, ebenso wie bestimmte Oberflächen in Bereichen mit hohem Publikumsverkehr, so z.B. in öffentlichen Verkehrsmitteln, Krankenhäusern. Telefonzellen. Schulen und insbesondere in öffentlichen Toiletten.

In der Alten- und Krankenpflege erfordern die häufig geminderten Abwehrkräfte der Betroffenen sorgfältige Maßnahmen gegen Infektionen, insbesondere auf Intensivstationen und in der häuslichen Pflege.

Besondere Sorgfalt bedarf die Verwendung medizinischer Gegenstände und Geräte bei medizinischen Untersuchungen, Behandlungen und Eingriffen, vor allem dann, wenn derartige Geräte oder Gegenstände mit lebendem Gewebe oder mit Körperflüssigkeiten in Kontakt kommen. Im Falle von Langzeit- oder Dauerkontakten, beispielsweise bei Implantaten. Kathetern, Stents, Herzklappen und Herzschrittmachern, können Bakterienkontaminationen zu einem lebensbedrohenden Risiko für den Patienten werden.

Es wurde bereits auf vielfältige Weise versucht, die Ansiedelung und Ausbreitung von Bakterien auf Oberflächen zu unterbinden. In J. Microbiol, Chemoth, **31** (1993), 261-271 beschreiben S.E.Tebbs und T.S.J.Elliott lackartige Beschichtungen mit quaternären Ammoniumsalzen als antimikrobiell wirkenden Komponenten. Es ist bekannt. daß diese Salze von Wasser, wäßrigen oder anderen polaren Medien sowie von Körperflüssigkeiten aus dem Beschichtungsmaterial herausgelöst werden und ihre Wirkung somit nur von kurzer Dauer ist. Dies gilt gleichermaßen für die Einarbeitung von Silbersalzen in Beschichtungen, so beschrieben in WO 92/18098.

T. Ouchi und Y. Ohya beschreiben in Progr.Polym.Sci. **20** (1995), 211 ff., die Immobilisierung von bakteriziden Wirkstoffen auf Polymeroberflächen durch kovalente Bindung oder ionische Wechselwirkungen. Häufig sind in solchen Fällen die keimtötenden Wirkungen gegenüber dem reinen Wirkstoff deutlich reduziert. Heteropolare Bindungen erweisen sich oft als nicht hinreichend stabil. Darüber hinaus führt die Keimabtötung in der Regel zu unerwünschten Ablagerungen auf den Oberflächen, die die weitere bakterizide Wirkung maskieren und die Grundlage für eine nachfolgende Bakterienbesiedelung bilden.

W.Kohnen et al. berichten in ZBl.Bakt.Suppl.26. Gustav Fischer Verlag, Stuttgart-Jena-New York, 1994, Seiten 408 bis 410, daß die Adhäsion von Staphylococcus epidermidis auf einem Polyurethanfilm vermindert wird, wenn der Film durch eine Glimmentladung in Gegenwart von Sauerstoff vorbehandelt und dann mit Acrylsäure gepfropft wird.

In der Literatur wird weiterhin die Pfropfung von Monomeren auf Substrate beschrieben, auf die zuvor ein Peroxid-haltiges Polymer als Makroinitiator aufgebracht wurde. In diesen Systemen befinden sich die photolytisch oder thermisch spaltbaren Gruppen im Polymergerüst (DE 30 44 531, EP 0 370 477). Bei der Spaltung dieser Gruppen unter Bildung von Radikalen wird das Polymergerüst des Makroinitiators zerteilt und verliert damit seine Stabilität. Ein solches Polymer kann nicht unter Bildung eines dauerhaften "interpenetrierenden Netzwerks'' (IPN) auf andere Substrate aufgebracht werden, da durch die Spaltung der obengenannten Gruppen kleine, aus dem Substratnetzwerk ausdiffundierende Polymerstücke gebildet wer-den.

In der DE-OS 22 42 818 wird die Herstellung von Polymeren mit in Seitenketten vorliegenden Peroxydiestergruppen erwähnt, für die jedoch keine spezielle Anwendung genannt wird.

Wie erwähnt, ist es wichtig, daß bei Verwendung medizinischer Gegenstände und Geräte bei medizinischen Untersuchungen, Behandlungen und Eingriffen eine Bakterienkontamination dieser Gegenstände und Geräte verhindert wird. Bei manchen dieser Gegenstände und Geräte, die mittel- oder langfristig mit lebendem Gewebe oder Körperflüssigkeiten in Kontakt kommen, ist zudem eine Haftung und Ausbreitung von körpereigenen Zellen ausgesprochen unerwünscht. So sind Zellbesiedelungen bei mittelfristig intrakorporal applizierten Kathetern ebenso schädlich wie bei langfristig implantierten Stents oder Herzklappen.

Weiterhin kann die Transparenz von Intraokularlinsen infolge von Zellbesiedelung nach der Implantation kontinuierlich abnehmen. Eine Reihe von Verfahren zielt darauf ab, eine Zellbesiedelung, beispielsweise durch die Einarbeitung bestimmter Metalle oder Metallsalze in die Halterung der Intraokularlinse zu vermeiden, wobei die Wirkung jedoch meist unvollständig und nicht nachhaltig ist. Auch in WO 94/16648 wird ein Verfahren beschrieben, durch das die Proliferation von Zellen auf der Oberfläche von implantierten Okularlinsen aus Polymermaterial verhindert werden soll.

Gemäß EP 0 431 213 sollen Polymere mit zellabweisenden Eigenschaften ausgestattet werden, indem ihre Oberfläche mit starken Mineralsäuren hydrophiliert wird. Die nachträgliche chemische Modifikation von Polymeroberflächen ist jedoch meist nicht gleichmäßig. Es bleiben in der Regel nicht oder nicht ausreichend behandelte Stellen zurück, die Ausgangspunkte für eine Zellbesiedelung bilden. Weiterhin sind die zellabweisenden Eigenschaften der so behandelten Oberflächen häufig nicht dauerhaft.

Andererseits sind für bestimmte Verwendungen Gegenstände mit Ober flächen erwünscht, die bakterienabweisend sind, aber die Besiedelung mit Zellen fördern. Das gilt z.B. für eine Reihe von Geräten für medizinische Untersuchungen, Behandlungen und Eingriffe und ebenso für manche Prothesen, die in das Gewebe einwachsen sollen, in das sie implantiert wurden. Solche Geräte und Prothesen, z.B. künstliche Hüftgelenke oder Zähne, bestehen häufig aus Polymeren oder aus polymerbeschichteten anderen Materialien, wie Metallen.

Schließlich müssen Materialien für Geräte und Vorrichtungen, die mit Körperflüssigkeiten, wie Blut oder Lymphe, oder mit Gewebe in Kontakt kommen, verträglich für ihre fremde Umgebung sein. Insbesondere ist Blutverträglichkeit eine wichtige erwünschte Eigenschaft. Die Materialien müssen also möglichst lange Blutgerinnungszeiten haben, d.h. möglichst wenig ausgeprägte thrombogene Eigenschaften haben.

Es gibt also verschiedene, einander teilweise ausschließende Anforderungen an die bioaktiven Eigenschaften der Oberfläche von Polymeren, die für medizinische Verwendungen bestimmt sind. Sie sollen stets bakterienabweisend und verträglich mit Körperflüssigkeiten und Gewebe sein, aber wahlweise zellproliferationshemmend oder -fördernd wirken.

Der vorliegenden Erfindung lag die Aufgabe zugrunde, ein verbessertes Verfahren zur Beschichtung von Oberflächen zu entwickeln, mit dem die Oberflächen bioaktiv, nämlich bakterienabweisend und verträglich für Körperflüssigkeiten, insbesondere mit Blut, und Gewebe sowie wahlweise zellproliferationshemmend oder -fördernd beschichtet werden können, ohne daß die mechanischen Eigenschaften der behandelten Materialien dadurch verändert werden oder sonstige erhebliche Nachteile eintreten.

### 2. Kurzbeschreibung der Erfindung

Es wurde überraschenderweise gefunden. daß sich die Oberfläche von Substraten, insbesondere von polymeren Substraten, vorteilhaft bioaktiv beschichten läßt, wenn man mindestens ein Vinylmonomer der allgemeinen Formel 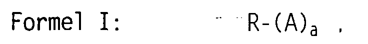 in der
- R: einen olefinisch ungesättigten organischen Rest, vorteilhaft einen Kohlenwasserstoffrest, mit der Wertigkeit a bedeutet,
- A: eine Carboxylgruppe -COOH, Schwefelsäuregruppe -OSO₂OH, Sulfonsäuregruppe -SO₃H, Phosphorsäuregruppe -OPO(OH)₂, Phosphonsäuregruppe -PO(OH)₂, Phosphorigsäuregruppe -OP(OH)₂, phenolische Hydroxylgruppe oder ein Salz einer der genannten Gruppen bezeichnet, und
- a: für eine ganze Zahl von 1 bis einschließlich 6, vorzugsweise für 1, 2 oder 3 steht,
strahleninduziert oder thermisch auf einer Substratoberfläche pfropfpolymerisiert, die zuvor mit einem Makroinitiator mit Radikale bildenden Gruppen in Seitenketten des Polymergerüstes behandelt wurde; mit der Maßgabe, daß ein Vinylmonomer I, in dem A die Carboxylgruppe -COOH oder ein Salz der Carboxylgruppe (also eine Carboxylatgruppe) bedeutet, entweder mindestens einen weiteren Rest A mit einer anderen der für A genannten Bedeutungen enthält oder zusammen mit mindestens einem weiteren Vinylmonomeren I verwendet wird, in dem A eine andere der für A genannten Bedeutungen hat.

Bei einer Ausführungsform der Erfindung wird neben einem bzw. anstatt eines einfach olefinisch ungesättigten Vinylmonomer(s) I ein vernetzendes Vinylmonomer (Vernetzer) mitverwendet bzw. verwendet, das mindestens 2, gegebenenfalls 3 oder 4 olefinische Doppelbindungen aufweist. Das vernetzende Vinylmonomer kann selbst Gruppen A enthalten, ist dann also ein Vinylmonomer I und selbst bioaktiv. In diesem Fall muß nicht, kann aber ein einfach olefinisch ungesättigtes Vinylmonomer I zusätzlich verwendet werden. Wenn jedoch das vernetzende Vinylmonomer frei von Gruppen A ist. muß zusätzlich ein einfach olefinisch ungesättigtes Vinylmonomer I eingesetzt werden. Der Vernetzer kann, gegebenenfalls zusammen mit einem einfach olefinisch ungesättigten Vinylmonomer I, auf das bereits mit dem Makroinitiator behandelte Substrat aufgebracht werden. Vorteilhaft fügt man ihn aber der Lösung des Makroinitiators zu, wenn das Substrat mit diesem behandelt wird.

Das gemeinsame Kennzeichen der Monomeren der Formel I ist, daß sie 1 oder 2 olefinische Doppelbindungen sowie mindestens eine saure Gruppe oder ein Salz einer sauren Gruppe aufweisen. Unter den Salzen werden die Alkalisalze und insbesondere die Natriumsalze bevorzugt. Saure Gruppen, wie Carboxyl- und Sulfonsäuregruppen, können durch Neutralisieren (z.B. mit Natriumhydroxyd oder in Phosphatpuffern) nach der Pfropfung in Carboxylat- bzw. Sulfonatgruppen überführt und Carboxylatgruppen durch Ansäuern zu Carboxylgruppen werden. In den letzteren Fällen wird also eine bestimmte Gruppe A in eine andere Gruppe A umgewandelt.

### 3. Vorteile der Erfindung

Überraschenderweise sind die bakterienabweisenden Eigenschaften der erfindungsgemäß mit einem carboxyl- oder carboxylatgruppenhaltigen Monomeren I zusammen mit einem anderen Monomeren I beschichteten Oberflächen deutlich stärker ausgeprägt als dies bei der Modifizierung mit Acrylsäure nach W. Kohnen et al., loc. cit., unter vergleichbaren Bedingungen der Fall ist.

Die erfindungsgemäß beschichteten Oberflächen zeigen eine bemerkenswerte Kombination vorteilhafter Eigenschaften und daher eine hervorragende physiologische Verträglichkeit. Sie sind insbesondere gut blutverträglich und vermindern die Adhäsion und Vermehrung von Bakterien in einem hohen Maße auch über längere Zeit. Von dieser Wirkung betroffene Bakterien sind u.a. Staphylococcus aureus, Staphylococcus epidermidis, Streptococcus pyogenes, Klebsiella pneumoniae, Pseudomonas aeruginosa und Escherichia coli. Gleichzeitig wird meistens auch die Proliferation von Zellen inhibiert, beispielsweise von Fibroblasten und Endothelzellen, wie menschlichen Nabelschnurzellen. Die besonderen Bedingungen, unter denen eine Beschichtung bakterienabweisend, aber zellproliferationsfördernd sind, werden später erläutert. Die Oberflächen der erfindungsgemäß beschichteten Substrate sind frei von migrationsfähigen und/oder extrahierbaren Monomer- und Oligomeranteilen. Unerwünschte Nebenwirkungen durch freigesetzte körperfremde Stoffe oder durch abgetötete Bakterien werden von vornherein vermieden.

Die Mitverwendung eines Vernetzers führt zu besser haftenden, delaminationsstabileren Beschichtungen. Das gilt insbesondere dann, wenn der Vernetzer zusammen mit dem Makroinitiator bei der Behandlung des Substrats angewandt wird.

### ^{4. Beschreibung der Erfindung}

Bei dem erfindungsgemäßen Verfahren werden die Substratoberflächen zunächst, wie in der Folge näher beschrieben, mit einem Makroinitiator mit Radikale bildenden Gruppen in der Seitenkette behandelt und anschließend unter Einwirkung von UV-Licht oder Wärme durch schonende Pfropfpolymerisation oder -copolymerisation beschichtet.

### 4.1 Die Vinylmonomeren der Formel I

Die Pfropf(co)polymerisation wird vorteilhaft mit polymerisierbaren Vinylmonomeren oder Mischungen von Vinylmonomeren der allgemeinen Formeln II oder III 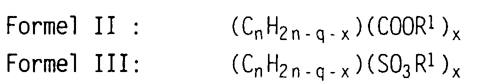 durchgeführt. In den Formeln, die in der allgemeinen Formel I eingeschlossen sind, steht
n jeweils unabhängig für eine ganze Zahl von 2 bis einschließlich 6;
x jeweils unabhängig für 1 oder 2:
q jeweils unabhängig für 0 oder, sofern n = 4, 5 oder 6 ist, für 2; und
   bedeutet der Rest R¹ jeweils unabhängig -H oder ein Äquivalent eines Metallions, insbesondere ein Alkalimetallion.

Den gegebenen Definitionen entsprechend bedeutet der Rest (CₙH_{2n-q-x})- jeweils unabhängig einen geradkettigen oder verzweigten einwertigen Alkenylrest (q=0, x=1) oder Alkadienylrest (q=2, x=1) oder einen zweiwertigen Alkenylenrest (q=0, x=2) oder Alkadienylenrest (q=2, x=2).

Anstelle von zwei Vinylonomeren II und III kann man auch nur ein Monomer (II + III) einsetzen, das die Gruppen -COOR¹ und -SO₃R¹ in demselben Molekül enthält.

Auch vom Benzol abgeleitete Vinylmonomere der allgemeinen Formel 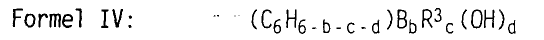 können eingesetzt werden, worin
B jeweils unabhängig einen ein- oder zweiwertigen geradkettigen oder verzweigten Rest der Formeln (CₙH_{2n-1-q-y})(COOR¹)_{y} oder (CₙH_{2n-1-q-y})(SO₃R¹)_{y} bedeutet, wobei R¹, n und q wie zuvor definiert sind und y für 0, 1 oder 2 steht;
R³ jeweils unabhängig C₁₋₄-Alkyl, -NH₂, -COOH, -SO₃H, -OSO₃H, -OPO(OH)₂, -PO(OH)₂, -OP(OH)₂. -OPO(O⁻)OCH₂-CH₂-N⁺(CH₃)₃, -PO(O⁻)O-CH₂-CH₂-N⁺(CH₃)₃, -OP(O⁻)OCH₂-CH₂ -N⁺(CH₃)₃ oder ein Salz, insbesondere ein Alkalisalz, der genannten sauren Gruppen bedeutet;
b für 1, 2, oder 3 steht;
c für 0, 1, 2, oder 3 steht; und
d für 0, 1, 2, oder 3 steht;
   mit der Maßgabe, daß b + c + d ≤ 6, vorteilhaft ≤4 ist.

Natürlich können auch beliebige Mischungen von Vinylmonomeren der allgemeinen Formeln II, III und IV für das erfindungsgemäße Verfahren eingesetzt werden.

Andere geeignete Vinylmonomere sind der Formel I entsprechende Schwefelsäuren und deren Salze; Sulfonsäuren und deren Salze; Phosphonsäuren und deren saure oder neutrale Salze, saure Phosphonsäureester und deren Salze; Phosphorsäuren und deren saure oder neutrale Salze, saure Phosphorsäureester und deren Salze; sowie Phosphorigsäuren und deren saure oder neutrale Salze, deren saure Ester und deren Salze. Auch diese Monomeren können im Gemisch untereinander und/oder mit den Monomeren der allgemeinen Formeln II, III und IV für das erfindungsgemäße Verfahren verwendet werden.

Schließlich seien noch 1 bis 3-wertige (oder -basische) Phenole sowie deren Salze, die der Formel I entsprechen, als geeignete Vinylmonomere erwähnt. Auch sie werden gegebenenfalls im Gemisch untereinander und/oder mit den zuvor erwähnten Vinylmonomeren eingesetzt.

Für das erfindungsgemäße Verfahren hat sich eine Kombination aus Vinylmonomeren I bewährt, die zu Beschichtungen führt, welche einerseits Carboxyl- und/oder Carboxylatgruppen und andererseits Sulfonsäure- und/oder Sulfonatgruppen aufweisen. Es gibt unter dem Aspekt der Verträglichkeit hinsichtlich der genannten Gruppen drei mögliche Zweierkombinationen, nämlich Carboxyl- und Sulfonsäuregruppen, Carboxyl- und Sulfonatgruppen sowie Carboxylat- und Sulfonatgruppen, weiterhin zwei Dreierkombinationen, nämlich Carboxyl-, Carboxylat- und Sulfonatgruppen sowie Carboxyl-, Sulfosäure- und Sulfonatgruppen. Alle diese Kombinationen charakterisieren brauchbare Ausführungsformen des erfindungsgemäßen Verfahrens.

In der genannten Kombination kann das molare Verhältnis von Carboxyl- und/oder Carboxylatgruppen zu Sulfonsäure- und/oder Sulfonatgruppen in der Beschichtung in weiten Grenzen schwanken. Besonders ausgeprägte zellproliferationsinhibierende Eigenschaften werden erzielt, wenn das genannte Verhältnis 0.2 bis 3, vorteilhaft 0.4 bis 3 und insbesondere 0.4 bis 2 beträgt. Die beschichteten Oberflächen zeigen in bemerkenswerter Weise bakterienabweisende, aber zellproliferationsfördernde Eigenschaften, wenn das besagte molare Verhältnis 2 bis 10, vorteilhaft 3 bis 10 und insbesondere 3 bis 5 beträgt. Zellproliferationsfördernd im Sinne der Erfindung ist eine Beschichtung dann, wenn die Haftung und Vermehrung von Säugetierzellen durch die Beschichtung im Vergleich zu der unbeschichteten Oberfläche verbessert oder jedenfalls weniger stark beeinträchtigt wird als die Haftung und Vermehrung von Bakterien.

Von den geeigneten Monomeren der allgemeinen Formel I, die eine oder mehrere gleiche oder verschiedene Reste A im Molekül enthalten, seien beispielsweise genannt;
Acrylsäure, Natriumacrylat, 4-Vinylsalicylsäure, Itaconsäure, Vinylessigsäure, Zimtsäure, 4-Vinylbenzoesäure, 2-Vinylbenzoesäure, Sorbinsäure, Kaffeesäure, Maleinsäure, Methylmaleinsäure, Crotonsäure, Isocrotonsäure, Fumarsäure, Methylfumarsäure, Dimethylfumarsäure, Dihydroxymaleinsäure, Allylessigsäure; Allylschwefelsäure, Allylsulfonsäure, Methallylschwefelsäure, Methallylsulfonsäure, Natriumallylsulfat, Natriumallylsulfonat, Natriummethallylsulfat Natriummethallylsulfonat, Natriumvinylsulfonat, Vinylsulfonsäure, 4-Vinylbenzolsulfonsäure, 2- oder 4-Styrolsulfonsäure, Natrium-2- oder 4-styrolsulfonat, Natriumvinyltoluolsulfonat; Buten-(2)-diol-(1,4)-diphosphat, Buten-(2)-diol-(1,4)-diphosphonat, die Dinatriumsalze des Diphosphats bzw. Diphosphonats, 2-Vinylphenol, 2-Allylhydrochinon, 4-Vinylresorcin, m-Hydroxystyrol, o-Hydroxystyrol, p-Hydroxystyrol, Carboxyl-vinylbenzolsulfonsäure.

Bei einer weiteren Ausführungsform des erfindungsgemäßen Verfahrens verwendet man als Monomer I eine Mischung aus Monomeren der allgemeinen **Formeln V** und **VI**

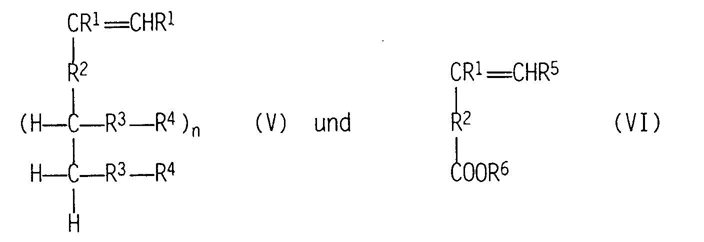

In den Formeln I und II bedeuten
R¹ Wasserstoff oder den Methylrest,
R² einen zweiwertigen organischen Rest, vorzugsweise einen aliphatischen, cycloaliphatischen oder aromatischen Kohlenwasserstoffrest mit bis zu 10 Kohlenstoffatomen, oder eine C-C-Einfachbindung.
R³ -O- oder -NH-,
R⁴ Wasserstoff oder den Rest -SO₃⁻Na⁺,
R⁵ Wasserstoff, den Methylrest oder den Rest -R²-COO⁻Na⁺.
R⁶ Wasserstoff oder Na und
n 4 oder 5;
mit der Maßgabe, daß mindestens einer der Substituenten R⁴ ein Rest -SO₃⁻Na⁺ ist.

In bevorzugten Monomeren V und VI bedeutet
R¹ Wasserstoff,
R² einen Alkylenrest mit 1 bis 4 Kohlenstoffatomen, einen Phenylenrest oder eine C-C-Einfachbindung.
R³ -O- oder -NH-,
R⁴ Wasserstoff oder den Rest -SO₃⁻Na⁺,
R⁵ Wasserstoff oder den Rest -R²-COO⁻Na⁺ ,
R⁶ Wasserstoff oder Na und steht
n für 4.

Die Monomeren V enthalten modifizierte Zuckerreste, vorzugsweise von Pentosen und insbesondere von Arabinose. Die Zuckerreste enthalten mindestens einen der Reste -O-SO₃⁻Na⁺ (O-Sulfat) oder -NH-SO₃⁻Na⁺ (N-Sulfat), bevorzugt benachbart zu dem Rest R². Sie weisen vorzugsweise 1 bis 4 dieser Reste auf. In einem Zuckerrest können O-Sulfat- und N-Sulfatreste gleichzeitig vorliegen, wobei dann der N-Sulfatrest bevorzugt benachbart zum Rest R² positioniert ist. Alternativ kann der Zuckerrest aber auch ausschließlich eine Art dieser Reste enthalten, z.B. nur O-Sulfatreste. Jede der genannten Spezies (nur O-Sulfat enthaltende sowie N-Sulfat-haltige Reste) ist für sich allein oder zusammen mit der anderen Spezies als Monomer V geeignet. Das Mischungsverhältnis beträgt also 0:100 bis 100:0.

Das Mengenverhältnis, in dem die Monomeren V und VI eingesetzt werden kann, in weiten Grenzen schwanken. So kann das Molverhältnis der N-Sulfat- und/oder O-Sulfatgruppen des Monomers V zu den Carboxyl- und/oder Carboxylatgruppen des Monomers VI bespielsweise 1:100 bis 100:1 betragen. Bevorzugte Molverhältnisse liegen zwischen 1:20 und 20:1.

Die Herstellung der Monomeren V ist in der deutschen Patentanmeldung 197 20 369.8 (O.Z. 5195) im einzelnen beschrieben.

Bei den Monomeren VI handelt es sich um bekannte und gut zugängliche Stoffe, die die für die heparinanaloge Wirkung erforderlichen Carboxyl- oder Carboxylatgruppen beisteuern. Geeignete Monomere VI haben eine olefinische Doppelbindung und eine oder zwei Carboxyl- bzw. Carboxylatfunktionen oder Funktionen, die in Carboxyl- bzw. Carboxylatfunktionen umgewandelt werden können, wie Carbonester-, Carbonamid- oder Carbonsäureanhydridgruppen. Als Gegenionen zur Carboxylatfunktion dienen Natriumionen. Beispiele für geeignete Monomere VI sind die Säuren (Meth)acrylsäure, Crotonsäure, 4-Vinylbenzoesäure, Maleinsäure, Fumarsäure, Itaconsäure, Vinylessigsäure, Zimtsäure, Crotonsäure, Isocrotonsäure, Methylmaleinsäure, Dimethylfumarsäure, Methylfumarsäure, Dihydroxymaleinsäure, Allylessigsäure sowie deren Natriumsalze.

### 4.2 Andere Vinylmonomere

Natürlich ist es auch möglich, anstelle oder neben Vinylmonomeren I andere Vinylmonomere einzusetzen, deren funktionelle Gruppen nach der Pfropfpolymerisation verändert werden. So kann man z.B. Carbonamidgruppen nachträglich durch Hydrolyse in saurem Medium in Carboxylgruppen umwandeln. Weiterhin kann man Carbonester- und Sulfonsäureestergruppen durch Hydrolyse in alkalischem Medium in Carboxylat- bzw. Sulfonatgruppen überführen. Beispiele für andere Vinylmonomere mit in Gruppen A umwandelbaren funktionellen Gruppen sind die Ester. Amide und Nitrile der unter 4.1 beispielhaft aufgeführten Säuren, insbesondere mit Alkanolen mit 1 bis 8 Kohlenstoffatomen, sowie Acrylnitril und Methacrylnitril.

Weiterhin kann man auch Vinylmonomere mitverwenden` die keine Gruppen A und keine in Gruppen A umwandelbare Gruppen enthalten, also hinsichtlich der biologischen Wirkung neutral oder allenfalls schwach wirksam sind. Dazu gehören z.B. Vinylester, wie Vinylacetat und Vinylpropionat; Vinylketone, wie Vinylethylketon; Olefine, wie 1-Buten, 1-Hexen und 1-Octen; Vinylaromaten, wie Styrol, Vinyltoluol und Divinylbenzol; und Vinylsiloxane.

Die anderen Monomeren können sogar in überwiegender Menge vorhanden sein. z.B. bis zu 90 mol% ausmachen.

### 2. Vernetzende Vinylmonomere (Vernetzer)

Die vernetzenden Vinylmonomeren weisen mindestens 2, gegebenenfalls 3 oder 4 olefinische Doppelbindungen auf. Ihre Mitverwendung führt einerseits zu dickeren und delaminationsstabileren Schichten und setzt andererseits die Bakterienadhäsion und -vermehrung unter sonst gleichen Bedingungen noch einmal erheblich herab. Vernetzer mit 3 oder 4 olefinischen Gruppen sind insoweit deutlich wirksamer als Diolefine. Diese führen zu zweidimensionalen Netzwerken. während die bevorzugten höherfunktionellen Vinylmonomeren dreidimensionale Netzwerke ergeben. Natürlich kann man auch mit zwei oder mehr verschiedenen Vernetzern arbeiten.

Wie bereits erwähnt, kann das vernetzende Vinylmonomer selbst Gruppen A enthalten, ist dann also ein Vinylmonomer I und trägt zur Bioaktivität der Beschichtung bei. Es kann aber auch in Gruppen A unwandelbare Funktionalität enthalten oder frei von funktionellen Gruppen sein. Die Mitverwendung insbesondere von Vernetzern mit 3 oder 4 olefinischen Doppelbindungen empfiehlt sich auch dann, wenn ein Vinylmonomer I mit 2 olefinischen Doppelbindungen zur Pfropfung eingesetzt wird.

Zweckmäßig enthalten die vernetzenden Vinylmonomeren hydrophile Gruppen, z.B. Hydroxylgruppen und/oder Alkylenoxidgruppen, und sind dann gleichzeitig hydrophile und vernetzende Vinylmonomere. Wenn man den Vernetzer der Lösung des Makroinitiators zur Behandlung des Substrats zusetzt, wendet man ihn zweckmäßige in Mengen von 0,01 bis 300 Molprozent, vorteilhaft von 10 bis 150 Molprozent an, bezogen auf die Peroxygruppen des Makroinitiators. Wenn man den Vernetzer zusammen mit den monoolefinisch ungesättigten Vinylmonomeren I anwendet. setzt man ihn zweckmäßig in Mengen von 0,1 bis 50 Molprozent, vorteilhaft von 1 bis 20 Molprozent ein, bezogen auf die monoolefinisch ungesättigten Vinylmonomeren I.

Geeignete vernetzende Vinylmonomere sind z.B. niedere Diolefine, wie 1,3-Butadien und Isopren; (Meth)acrylsäurederivate, wie Methylenbisacrylamid (MBAA), Ethylenglykoldimethacrylat (EGDMA), Diethylenglykoldimethacrylat, Ethylenglykoldiacrylat (EGDA), Diethylenglykoldiacrylat (DEGDA), Tetraethylenglykoldiacrylat oder -methacrylat, Polyethylenglykol-400-diacrylat, Polyethylenglykol-600-diacrylat, Polyethylenglykol-1000-diacrylat, Trimethylolpropantriacrylat, Trimethylolpropantrimethacrylat und Pentaerythrittetraacrylat; Polyvinylether, wie Diethylenglykoldivinylether, Polyethylenglykol-300-divinylether, Polyethylenglykol-1500-divinylether, Polyethylenglykol-6000-divinylether, Cyclohexan-1,4-dimethanoldivi-nylether, 1,4-Hexandioldivinylether, Glycerin-12EO-trivinylether und Pentaerythrit-64EO-tetravinylether; Kohlenhydratderivate, wie acryloylierte Hydroxypropylzellulose mit mehr als einer Acryloylgruppe pro Molekül; und Allylverbindungen, wie Allycinnamat, Tetraethylenglykoldiallylether und Pentaerythrittetraallylether.

### 3. Die Substratmaterialien

Als Substratmaterialien eignen sich besonders alle polymeren Kunststoffe, wie Polyurethane, Polyamide, Polyester und -ether, Polyetherblockamide, Polystyrol, Polyvinylchlorid, Polycarbonate, Polyorganosiloxane, Polyolefine, Polysulfone, Polyisopren, PolyChloropren, Polytetrafluorethylen (PTFE), entsprechende Copolymere und Blends sowie natürliche und synthetische Kautschuke, mit oder ohne strahlungssensitive Gruppen. Das erfindungsgemäße Verfahren läßt sich auch auf Oberflächen von lackierten oder anderweitig mit Kunststoff beschichteten Metall-, Glas- oder Holzkörpern anwenden.

Die polymeren Substrate können mannigfache Formen aufweisen, beispielsweise als Platten, Folien, Rohre oder Schläuche vorliegen, je nach der beabsichtigten Verwendung des betreffenden Erzeugnisses.

### 3. Der Makroinitiator

Geeignete Makroinitiatoren enthalten in Seitenketten photolytisch oder thermisch aktivierbare. Radikale bildende Gruppen, beispielsweise Peroxid-, Hydroperoxid-, Perester- oder Azogruppen. Ihre Synthese erfolgt beispielsweise durch polymeranaloge Reaktion von Carboxylgruppen-haltigen Polymeren mit Peroxyalkoholen oder mit Wasserstoffperoxid und anschließend mit Carbonsäurechloriden. Eine andere Synthese geht von Hydroxylgruppen-haltigen Polymeren aus, die mit Hydrazin umgesetzt und anschließend zur Azoverbindung oxidiert werden. Als Polymere eignen sich besonders solche, die aus Monomeren hergestellt werden, welche einerseits funktionelle Gruppen enthalten, die eine Anbindung an oder eine Umwandlung in eine photolytisch oder thermisch aktivierbare, Radikale bildende Gruppe gestatten und welche andererseits homo- oder copolymerisierbar, homo- oder cokondensierbar oder homo- oder coaddierbar sind. Bevorzugte Polymere sind die durch Polymerisation oder Copolymerisation erhältlichen Polymeren oder Copolymeren mit Kohlenstoffgerüst. Wenn Comonomere mitverwendet werden, können die Copolymeren Blockcopolymere oder Copolymere mit statistischer oder alternierender Abfolge der Monomeren sein.

Zu den geeigneten polymerisierbaren Monomeren zählen solche mit Hydroxylgruppen, wie Hydroxyalkyl(meth)acrylate, z.B. Hydroxyethylmethacrylat, 3-Hydroxypropylacrylat, 3-Hydroxypropylmethacrylat oder 4-Hydroxybutylacrylat; 2-Hydroxystyrol, 3-Hydroxystyrol, 4-Hy-droxystyrol, 4-Vinylresorcin, Allylalkohol, 2,3-Butadien-1-ol, 2-Buten-1,4-diol, 2-Buten-1-ol und 3-Methallylalkohol, Weiterhin geeignet sind Monomere mit Carboxylgruppen, wie Acrylsäure, Methacrylsäure, Maleinsäure, Dihydroxymaleinsäure, Fumarsäure und 4-Vinylbenzoesäure; mit Epoxy-Gruppen, wie Glycidyl(meth)acrylat; mit Anhydridgruppen, wie Maleinsäureanhydrid; oder mit Imidgruppen, wie Maleinsäureimid.

### 4. Behandlung des polymeren Substrats mit dem Makroinitiator

Zunächst wird der Makroinitiator, gegebenenfalls zusammen mit einem Vernetzer, in einem organischen Lösemittel gelöst. Geeignete Lösemittel sind z.B. Alkohole, wie Methanol, Ethanol, Propanol und Isopropanol; Ether, wie Diethylether, Tetrahydrofuran und Dioxan; Ester, wie Ethylacetat; Ketone, wie Aceton, Methylethylketon und Cyclohexanon; Kohlenwasserstoffe, wie Pentan, Hexan, Cyclohexan. Testbenzin, Benzol, Toluol der Xylol; Carbonsäuren, wie Ameisensäure und Essigsäure; halogenierte Kohlenwasserstoffe, wie Dichlormethan, Trichlormethan und Tetrachlormethan; oder stark polare Lösemittel, wie Dimethylformamid, Dimethylacetamid und Dimethylsulfoxid, Selbstverständlich kann man auch homogene Gemische aus zwei oder mehr der genannten Lösemittel einsetzen. Das Lösemittel sollte einerseits nach der Behandlung des Substrats möglichst rasch verdampfen, aber auch das Substrat anquellen. um ein Eindringen des Makroinitiators in die (oder eine "Legierung" mit der) Struktur des Substrats zu ermöglichen. Ein günstiges Lösemittel für eine gegebene Kombination von Makroinitiator und polymerem Substrat läßt sich durch orientierende Versuche unschwer ermitteln.

Die Konzentration des Makroinitiators in der Lösung beträgt zweckmäßig 0.5 bis 60 Gew.-%, insbesondere 1 bis 10 Gew.-%, Die Konzentration des gegebenenfalls mitverwendeten Venetzers liegt im allgemeinen in denselben Bereichen. Die letztgenannten Konzentrationen haben sich in der Praxis besonders bewährt und ergeben im allgemeinen in einem Durchgang zusammenhängende, das Substrat vollständig bedeckende Schichten aus dem Makroinitiator und gegebenenfalls einem Vernetzer mit Schichtdicken im nm-Bereich. Das Substrat wird mit der Lösung z.B. durch Tauchen. Streichen oder Besprühen behandelt und anschließend getrocknet, je nach Lösemittel und thermischer Empfindlichkeit des Makroinitiators bei Raumtemperatur oder leicht erhöhter Temperatur mit oder ohne Vakuum.

### 5. Beschichtung mit Vinylmonomeren

### 5.1 Thermische Pfropfung

Auf das mit dem Makroinitiator behandelte Polymersubstrat wird das Vinylmonomer oder werden die Vinylmonomeren, gegebenenfalls zusammen mit einem Vernetzer, aufgebracht, zweckmäßig in gelöster Form, und wiederum z.B. durch Tauchen. Streichen oder Sprühen. Das Monomer-beschichtete Substrat wird dann, je nach Makroinitiator, unter Ausschluß von Luftsauerstoff auf 30 bis 100°C, insbesondere auf 50 bis 90°C erhitzt, wodurch die Pfropfpolymerisation ausgelöst wird. Alternativ kann man das Substrat mit dem Makroinitiator auch in das/die Vinylmonomer(en) bzw. in dessen/deren Lösung eintauchen und im getauchten Zustand auf die genannten Temperaturen erhitzen. Geeignete Lösemittel sind, je nach dem Vinylmonomer, z.B. Wasser oder wäßrige Alkoholgemische, doch sind auch andere Lösemittel oder Lösemittelgemische verwendbar, sofern sie ein ausreichendes Lösevermögen für das Vinylmonomer und gegebenenfalls den Vernetzer besitzen, Je nach Löslichkeit der Monomeren und nach gewünschter Schichtdicke können die Konzentrationen der Vinylmonomeren und gegebenenfalls der Vernetzer in der Lösung zusammen 0,1 bis 40 Gew.-%, vorzugsweise 1 bis 10 Gew.-% betragen. Die letztgenannten Konzentrationen haben sich besonders bewährt und ergeben im allgemeinen in einem Durchgang zusammenhängende, den Makroinitiator vollständig bedeckende Schichten.

### 5.2 Photoinitiierte Pfropfung

Als Variante zur thermischen Pfropfung der Vinylmonomeren ist auch eine photoinitiierte Pfropfung möglich. Dazu werden die Vinylmonomeren, gegebenenfalls zusammen mit einem Vernetzer, auf das mit dem Makroinitiator behandelte Substrat aufgebracht, wie beschrieben. und die beschichteten Substrate werden dann bestrahlt. Alternativ können die Substrate auch im getauchten Zustand bestrahlt werden.

Die Temperaturen während der Bestrahlung betragen in beiden Fällen zweckmäßig 20 bis 60°C.

Die Bestrahlung erfolgt zweckmäßig mit Strahlen im kurzwelligen Segment des sichtbaren oder oder im langwelligen Segment des UV-Bereiches der elektromagnetischen Strahlung. Gut geeignet sind Strahlen mit Wellenlängen von 260 bis 500 nm, vorzugsweise von 290 bis 320 nm. Strahlen im genannten Wellenlängenbereich sind relativ weich und selektiv bezüglich der Polymerisation, greifen also weder das Substratpolymer noch das Gerüst des Makroinitiators an. Besonders geeignete Strahlungsquellen sind Excimer-UV-Strahler (Fa. Heraeus. D-63801 Kleinostheim) mit kontinuierlicher Strahlung, z.B. mit XeCl oder XeF als Strahlermedium. Im Prinzip sind auch Quecksilberdampflampen mit breitem UV-Spektrum und Strahlungsanteilen im sichtbaren bzw. im obengenannten Bereich brauchbar. Die Expositionszeiten betragen im allgemeinen 10 Sekunden bis 20 Minuten, vorzugsweise 2 bis 15 Minuten bei Strahlungsintensitäten im Bereich von 30 bis 200 mW/cm².

Bisweilen ist es zweckmäßig die beschriebenen Arbeitsschritte einschließlich der Behandlung mit dem Makroinitiator zu wiederholen. um mittels einer solchen Mehrschichttechnik eine hermetisch geschlossene und/oder dickere Beschichtung sicherzustellen.

### 6. Verwendung der beschichteten Substrate

Das erfindungsgemäße Verfahren gestattet die genaue Einstellung von Molverhältnissen verschiedener funktioneller Gruppen, die zur Inhibierung der Bakterienadhäsion und/oder -ausbreitung sowie für das gewünschte Verhalten der Oberfläche bezüglich der Zellproliferation optimal sind oder zu einer verbesserten Blutverträglichkeit führen. Erzeugnisse mit erfindungsgemäß beschichteter Oberfläche eigen sich zur Verwendung im medizinischen oder biotechnischen oder im Hygienebereich. Solche Erzeugnisse sind z.B. Folien, Schläuche, Rohrleitungen, Türgriffe und Toilettensitze. Speziell aus dem medizinischen Bereich seien Katheter, Implantate (wie Herzklappen und Stents), Blutbeutel, Wundverbände, Schläuche (z.B. für Drainagen), Membranen und Kontaktlinsen genannt.

Zur weiteren Erläuterung der vorliegenden Erfindung werden die folgenden Beispiele gegeben. Die darin verwendeten Vinylmonomeren sind stellvertretend für eine Vielzahl anderer unter die allgemeinen Formeln I bis VI fallenden geeigneten Verbindungen.

### Beispiele

### Eingesetzte Polymersubstrate

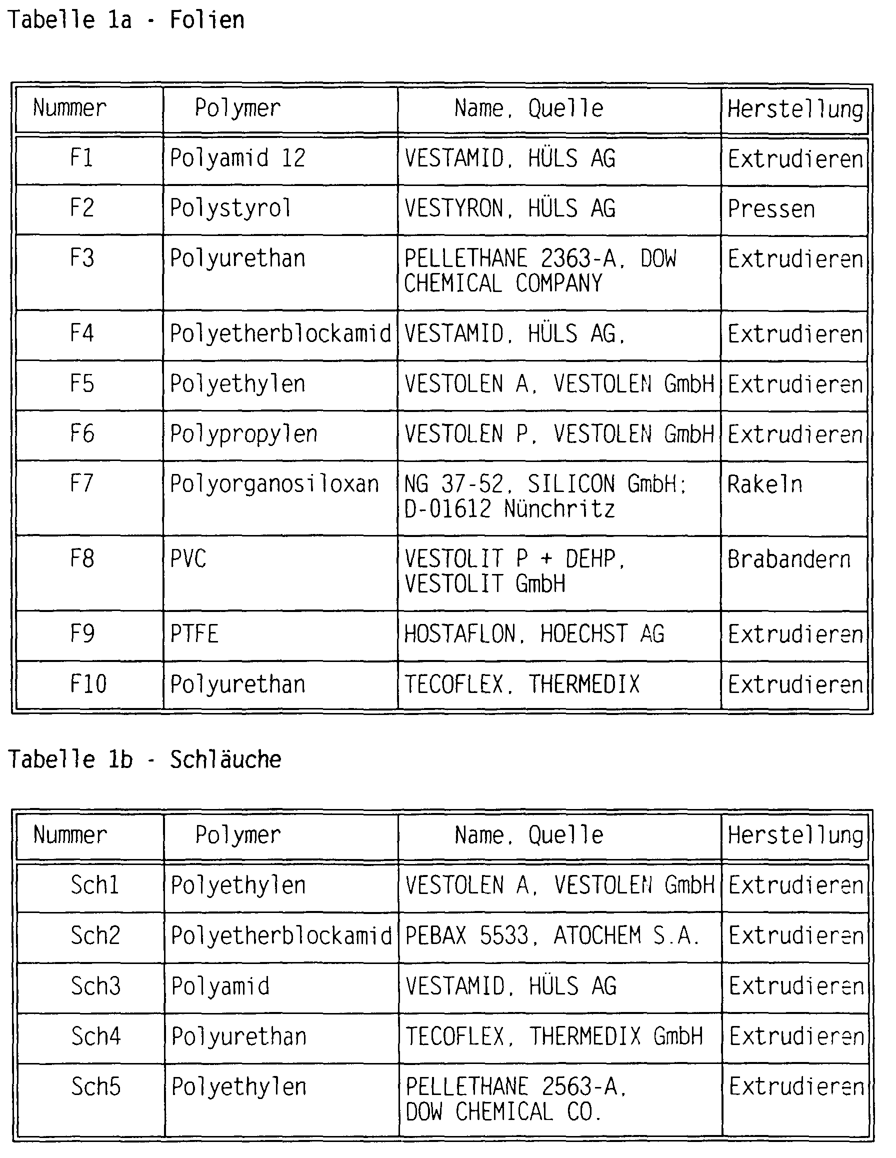

### Die verwendeten Makroinitiatoren

Als Makroinitiatoren werden Polymere mit Peroxyestergruppen verwendet. Sie werden als 5 gew.%-ige Lösungen in den in Tabelle 2 angegebenen Lösemitteln angewandt.

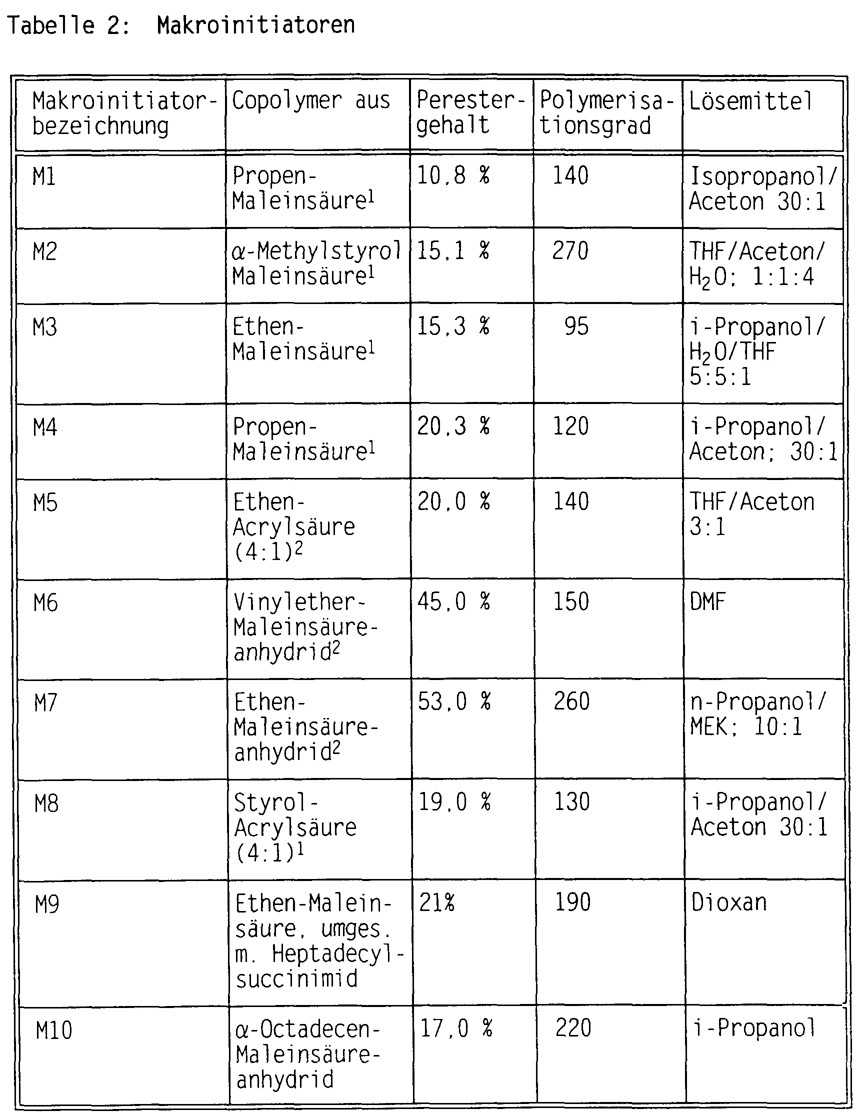

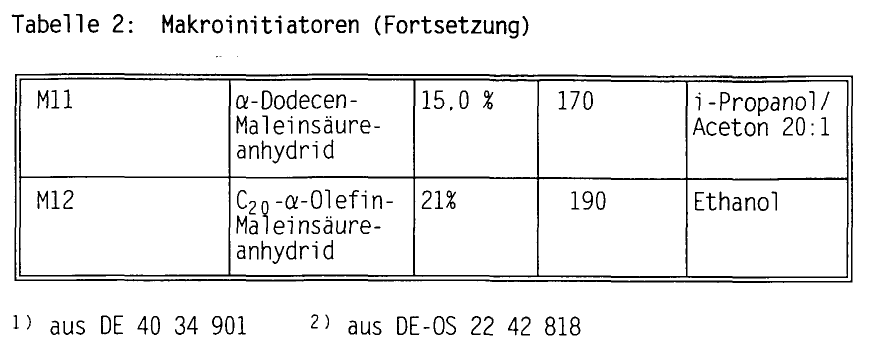

### Die verwendeten Vinylmonomeren

Von den in Tabelle 3 aufgeführten Monomeren wurden jeweils (a) 5 gew.-%ige wäßrige Lösungen, (b) 5 gew.-%ige Isopropanol-Lösungen oder (c) 5 gew.-%ige Aceton/THF/Wasser(4:4:1)-Lösungen hergestellt.

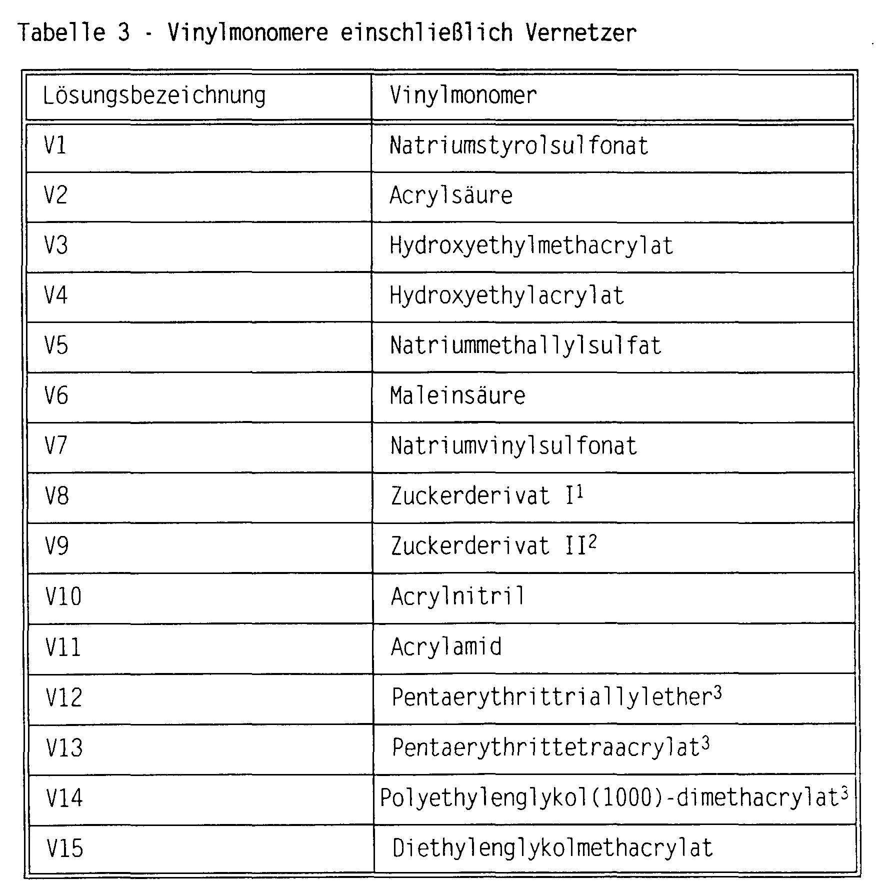

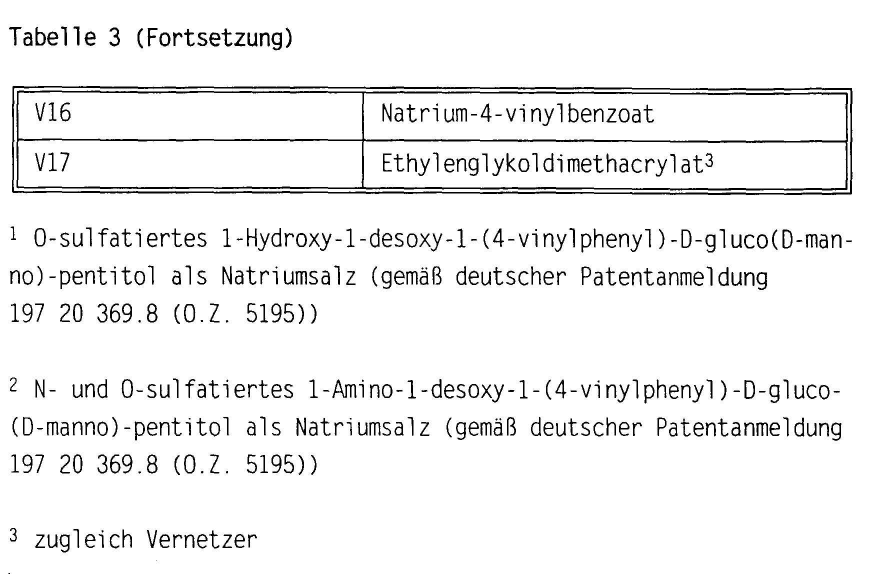

### Beschichtungsverfahren

Nach Fixierung des Makroinitiators und gegebenenfalls eines Vernetzers durch Tauchen in dessen bzw. deren Lösung und Trocknen an der Luft oder im Trockenschrank bei 50°C werden die Vinylmonomeren, gegebenenfalls zusammen mit einem Vernetzer, auf die Makroinitiatorschicht auf die in Tabelle 6 angegebene Weise aufgebracht und gehärtet.

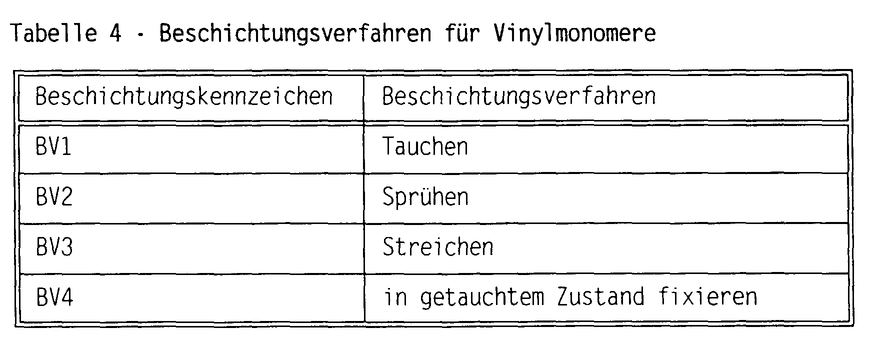

### Bestimmung der bakterienabweisenden Eigenschaften

Die Prüfung auf Adhäsion von Bakterien kann mit verschiedenen Stämmen vorgenommen werden. Hierzu eignen sich besonders die in Tabelle 5 aufgeführten Bakterien, da sie in klinischen Isolaten von infizierten Kathetern häufig vorkommen.

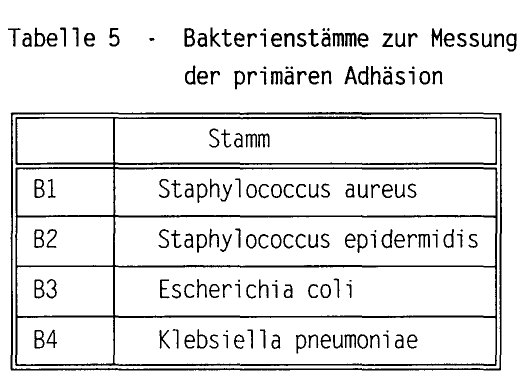

Das Verfahren zur Bestimmung der primären Adhäsion (also unabhängig von späterer Vermehrung) dieser Bakterienstämme wird beispielhaft für Klebsiella pneumoniae in der Folge beschrieben. Die primäre Adhäsion der anderen Stämme (B1 bis B3) wurde analog bestimmt.

### Bestimmung der primären Bakterienadhäsion unter statischen Bedingungen

Eine Über-Nacht-Kultur des Bakterienstammes Klebsiella pneumoniae in Hefeextrakt-Pepton-Glukose-Nährmedium (1% + 1% + 1%) wird abzentrifugiert und in Phosphat-gepufferter Saline (=PBS; 0,05m KH₂PO₄. pH 7,2 + 0,9 % NaCl) wieder aufgenommen. Man verdünnt mit PBS-Puffer auf ein Zellkonzentration von 10⁸ Zellen/ml. Die suspendierten Bakterien werden mit dem zu untersuchenden Folienstück für 3h in Berührung gebracht. Dazu werden doppelseitig beschichtete kreisförmige Folienstücke mit einem Durchmesser von 1,6 cm (=4,02 cm²) auf eine Präpariernadel gesteckt und mit der Zellsuspension geschüttelt. Einseitig beschichtete Folien werden in Form einer runden. ebenen Scheibe von 4,5 cm Durchmesser und mit einer Stützmembran aus 2-3 cm dickem Weich-PVC in eine Membranfilterapparatur eingespannt. Auf die nach oben zeigende Seite mit der zu prüfenden Beschichtung wird die Zellsuspension aufgegeben und 3h geschüttelt. Die Membranfilterapparatur muß dicht sein, d.h. es darf keine Zellsuspension durch undichte Zellen ausfließen.

Nach Ablauf der Kontaktzeit wird die Bakteriensuspension mit einer Wasserstrahlpumpe abgesaugt, und die Folienstücke werden zum Waschen mit 20 ml steriler PBS-Lösung in einem 100 ml Becherglas 2 min geschüttelt. Das Folienstück wird nochmals in sterile PBS-Lösung eingetaucht und dann in 10 ml erhitztem TRIS/EDTA (0,1M Tris-hydroxyethylaminomethan, 4 mM Ethylendiamintetraessigsäure, mit HCl auf pH 7,8 eingestellt) für 2 min im siedenden Wasserbad extrahiert.

Mit der Extraktionslösung werden kleine Eppendorf-Cups befüllt und sofort bis zur Biolumineszenz-Bestimmung des extrahierten Adenosintriphosphats (ATP) bei -20°C eingefroren. Die Bestimmung wird wie folgt ausgeführt: In ein transparentes Röhrchen aus Polycarbonat wird 100 µl Reagentienmix (Biolumineszenz-Test CLS II. Fa. BOEHRINGER MANNHEIM GmbH) gegeben, und über einen Zeitraum von 10 sec werden in einem Lichtimpuls-Meßgerät LUMAT LB9501 (Laboratorien Prof. Berthold GmbH. 75323 Bad Wildbad. Deutschland) die Lichtimpulse integriert. Dann wird eine 100 ul Probe zugegeben und erneut gemessen. Die relativen Lichteinheiten (RLU) werden durch Subtraktion der Lichtimpulse im Reagentienmix von der Anzahl der gemessenen Lichtimpulse im kompletten Ansatz erhalten. Dieser Wert steht in Relation zu der Anzahl der an der Folie adhärierten Bakterien. Der Umrechnungsfaktor zwischen dem RLU-Wert und der Bakterienzahl wird bestimmt, indem ein Aliquot von 0,1 ml der Bakteriensuspension mit 10⁸ Zellen/ml in 10 ml heißem TRIS/EDTA extrahiert und dann der ATP-Gehalt bestimmt wird.

### Ergebnisse

In der folgenden Tabelle 6 sind die verschiedenen Bedingungen und die Ergebnisse von insgesamt 20 Versuchen zusammengestellt.

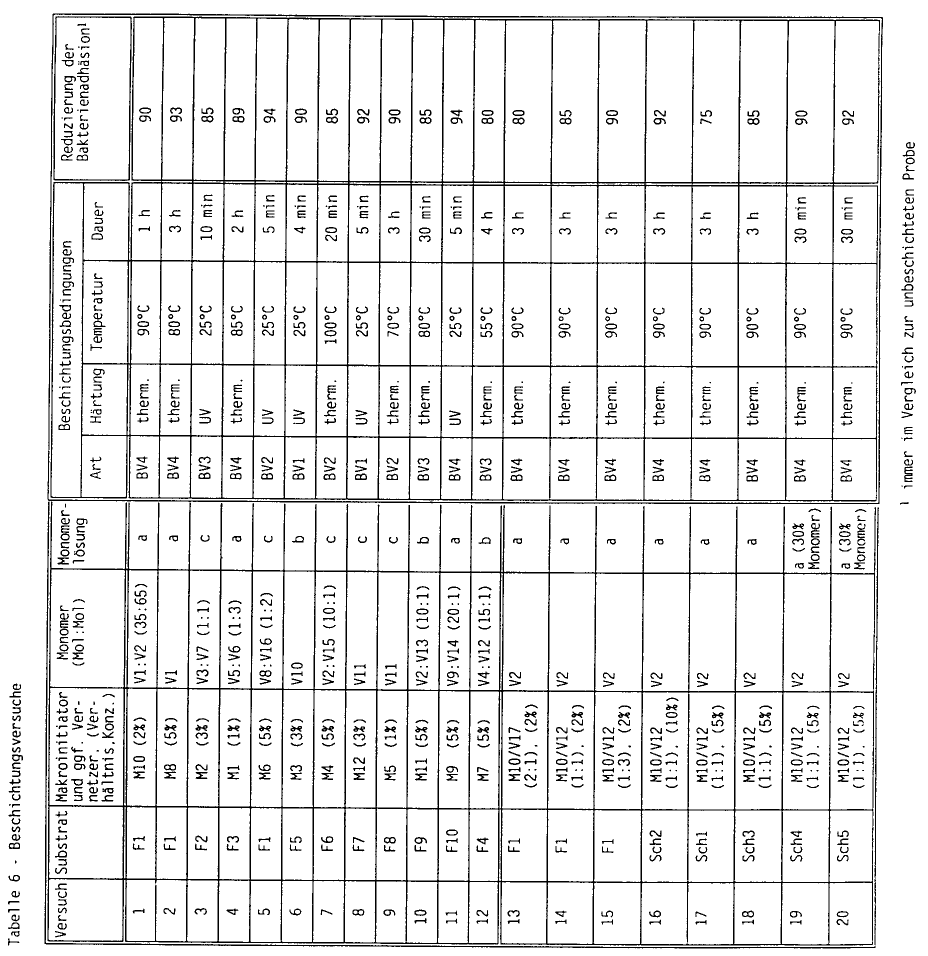

### Bestimmung der antithrombischen Wirksamkeit der im Versuch 5 beschichteten Polyamid 12-Folie

Die mit der partiellen Thromboplastinzeit (PTT) gemessene Blutgerinnungszeit wird durch gerinnungshemmende Stoffe, wie Heparin, beeinflußt. Zur Bestimmung der PTT wird Blutplasma mit einem Kontaktaktivator sowie Phospholipiden und Calciumionen versetzt, und damit eine Aktivierung der Kontaktaktivatoren ausgelöst sowie die Aktivierung des intrinsischen Gerinnungssystems katalysiert. Bei der praktischen Durchführung des Tests wird Blutplasma mit dem Kontaktaktivator (z.B. Kaolin) und Phospholipiden (z.B. Kephalin) versetzt und in Gegenwart der beschichteten PA 12-Folie bzw. zum Vergleich einer unbeschichteten PA 12-Folie 3 Minuten bei 37°C inkubiert. Dann wird Calciumchlorid zugesetzt und die Gerinnungszeit (PTT) gemessen.

Die PTT-Zeit der unbeschichteten PA 12-Folie liegt bei 35 sec. die der nach Versuch 5 beschichteten Folie bei 60 sec. Die Werte sind Mittelwerte aus Messungen mit Blutplasmen von drei Spendern.

Die Thrombinzeit (TZ) ist ein weiteres Maß für die gerinnungshemmende Wirkung eines Stoffes. In der Endphase der Gerinnung wird aus Fibrinogen das unlösliche Fibrin, ein weiches Gerinnsel, das sich durch kovalente Querverbindung in hartes Gerinnsel umwandelt. Diese Reaktion wird durch Thrombin katalysiert und durch gerinnungshemmende Stoffe, wie Heparin, verzögert. Zur Bestimmung der heparinanalogen Wirksamkeit wurde die TZ der beschichteten PA 12-Folie mit der TZ der unbeschichteten PA 12-Folie verglichen. TZ von Heparin in Abhängigkeit von der Dosis verglichen.

Die TZ der unbeschichteten PA 12-Folie betrug 21 sec, die der nach Versuch 5 beschichteten PA 12-Folie 26 sec.

## Patentansprüche

1. Verfahren zur bioaktiven Beschichtung der Oberfläche von Substraten, dadurch gekennzeichnet, daß man mindestens ein Vinylmonomer der allgemeinen Formel
R-(A)ₐ , Formel I
in der
R einen olefinisch ungesättigten organischen Rest mit der Wertigkeit a bedeutet,
A eine Carboxylgruppe -COOH, Schwefelsäuregruppe -OSO₂OH, Sulfonsäuregruppe -SO₃H, Phosphorsäuregruppe -OPO(OH)₂, Phosphonsäuregruppe -PO(OH)₂, Phosphorigsäuregruppe -OP(OH)₂, phenolische Hydroxylgruppe oder ein Salz einer der genannten Gruppen bezeichnet und
a für eine ganze Zahl von 1 bis einschließlich 6 steht.
strahleninduziert oder thermisch auf einer Substratoberfläche pfropfpolymerisiert, die zuvor mit einem Makroinitator mit Radikale bildenden Gruppen in Seitenketten des Polymergerüstes behandelt wurde; mit der Maßgabe, daß ein Monomer I, in dem A eine Carboxylgruppe -COOH oder ein Salz der Carboxylgruppe bedeutet, entweder mindestens einen weiteren Rest A mit einer anderen der für A genannten Bedeutungen enthält oder zusammen mit mindestens einem weiteren Monomeren I verwendet wird, in dem A eine andere der für A genannten Bedeutungen hat.

2. Verfahren nach Anspruch 1, dadurch gekennzeichnet, daß man ein vernetzendes Vinylmonomer mitverwendet, das mindestens 2 olefinische Doppelbindungen aufweist.

3. Verfahren nach Anspruch 1, dadurch gekennzeichnet, daß man ein vernetzendes Vinylmonomer mitverwendet, das 3 oder 4 olefinische Doppelbindungen aufweist.

4. Verfahren nach einem der Ansprüche 1 bis 3, dadurch gekennzeichnet, daß man das vernetzende Vinylmonomer zusammen mit dem Makroinitiator bei der Behandlung des Substrats anwendet.

5. Verfahren nach einem der Ansprüche 1 bis 3, dadurch gekennzeichnet, daß man das vernetzende Vinylmonomer, gegebenenfalls zusammen mit einem monoolefinisch ungesättigten Vinylmonomer I, auf das mit dem Makroinitiator behandelte Substrat aufbringt.

6. Verfahren nach einem der Ansprüche 1 bis 5, dadurch gekennzeichnet, daß man anstelle von oder neben Vinylmonomeren I andere Vinylmonomere mit funktionellen Gruppen einsetzt, die nach der Pfropfung in Gruppen A umgewandelt werden.

7. Verfahren nach einem der Ansprüche 1 bis 6, dadurch gekennzeichnet, daß man anstelle von oder neben Vinylmonomeren I andere Vinylmonomere mitverwendet, die keine Gruppen A und keine in Gruppen A unwandelbaren Gruppen enthalten.

8. Verfahren nach einem der Ansprüche 1 bis 7, dadurch gekennzeichnet, daß die Vinylmonomeren I so gewählt werden, daß die gepfropften Beschichtungen Carboxyl- und/oder Carboxylatgruppen sowie Sulfonsäure- und/oder Sulfonatgruppen enthalten.

9. Verfahren nach Anspruch 8, dadurch gekennzeichnet, daß das molare Verhältnis von Carboxyl- und/oder Carboxylatgruppen zu Sulfonsäure und/oder Sulfonatgruppen 0,2 bis 3 beträgt.

10. Verfahren nach Anspruch 8, dadurch gekennzeichnet, daß das molare Verhältnis von Carboxyl- und/oder Carboxylatgruppen zu Sulfonsäure und/oder Sulfonatgruppen 0,4 bis 3 beträgt.

11. Verfahren nach Anspruch 8, dadurch gekennzeichnet, daß das molare Verhältnis von Carboxyl- und/oder Carboxylatgruppen zu Sulfonsäure und/oder Sulfonatgruppen 0,4 bis 2 beträgt.

12. Verfahren nach Anspruch 8, dadurch gekennzeichnet, daß das molare Verhältnis von Carboxyl- und/oder Carboxylatgruppen zu Sulfonsäure und/oder Sulfonatgruppen 2 bis 10 beträgt.

13. Verfahren nach Anspruch 8, dadurch gekennzeichnet, daß das molare Verhältnis von Carboxyl- und/oder Carboxylatgruppen zu SulfonSäure und/oder Sulfonatgruppen 3 bis 10 beträgt.

14. Verfahren nach Anspruch 8, dadurch gekennzeichnet, daß das molare Verhältnis von Carboxyl- und/oder Carboxylatgruppen zu Sulfonsäure und/oder Sulfonatgruppen 3 bis 5 beträgt.

15. Verfahren nach einem der Ansprüche 1 bis 7, dadurch gekennzeichnet, daß das Vinylmonomer I so gewählt wird, daß die gepfropfte Polymerschicht Phosphorsäuregruppen oder Phosphonsäuregruppen oder deren Salze oder Ester enthält.

16. Verfahren nach einem der Ansprüche 1 bis 15, dadurch gekennzeichnet, daß man die Propfpolymerisation der Vinylmonomeren durch Strahlen im Bereich von 250 bis 500 nm bewirkt.

17. Verfahren nach einem der Ansprüche 1 bis 15, dadurch gekennzeichnet, daß man die Polymerisation der Monomeren durch UV-Strahlen im Bereich von 290 bis 320 nm bewirkt.

18. Verfahren nach einem der Ansprüche 1 bis 15, dadurch gekennzeichnet, daß man die Polymerisation thermisch initiiert.

19. Verfahren nach Anspruch 18, dadurch gekennzeichnet, daß die Temperatur 30 bis 100°C beträgt.

20. Verfahren nach einem der Ansprüche 1 bis 19, dadurch gekennzeichnet, daß die polymeren Substrate als Platten, Folien, Rohre oder Schläuche vorliegen.

21. Erzeugnisse mit gemäß dem Verfahren eines der Ansprüche 1 bis 20 ganz oder teilweise beschichteter Oberfläche zur Verwendung im medizinischen oder biotechnischen oder im Hygienebereich.

22. Erzeugnisse nach Anspruch 21, dadurch gekennzeichnet, daß die Erzeugnisse Katheter, Implantate, Blutbeutel, Wundverbände, Schläuche, Membranen oder Kontaktlinsen sind.

23. Verwendung der Erzeugnisse nach den Ansprüchen 21 und 22 für medizinische oder biotechnische Zwecke oder im Hygienebereich.
